# DEMANDE DE BREVET EUROPEEN

(11) **EP 3 643 228 A1**
(43) Date de publication de la demande: **29.04.2020**
(21) Numéro de dépôt: 19204484.0
(22) Date de dépôt: 22.10.2019
(51) Int. Cl.: A61B 5/04, A61B 5/0428, A61B 5/055, A61B 5/00

(54) **PROCÉDÉ DE CORRECTION EN TEMPS RÉEL D'AU MOINS UN SIGNAL ÉLECTRO-PHYSIOLOGIQUE**

(30) Priorité: 23.10.2018 FR 1859800
(71) Demandeur: Schiller Medical, 67160 Wissembourg (FR); Université de Lorraine, 54052 Nancy Cedex (FR); Centre Hospitalier Régional de Nancy, 54035 Nancy Cedex (FR)
(72) Inventeur: FELBLINGER, Jacques, 54850 Méréville (FR); PETITMANGIN, Grégory, 67480 Leutenheim (FR); ODILLE, Freddy, 54600 Villers-lès-Nancy (FR); DOS REIS SANCHEZ, Jesus Enrique, 54000 Nancy (FR)
(74) Mandataire: Cabinet Bleger-Rhein-Poupon

(57) **Abrégé**

L'invention concerne un procédé de correction en temps réel d'au moins un signal électro-physiologique mesuré sur un sujet en présence de variations d'au moins une caractéristique d'un environnement électromagnétique, lesdites variations générant des artéfacts dans ledit signal électro-physiologique. Ce procédé comporte les étapes suivantes :
- filtrage passe-bas du signal mesuré avec une première fréquence de coupure fc₁ supérieure à la fréquence des artéfacts ;
- amplification du signal avec une dynamique d'entrée basée sur une tension égale à au moins 80% de la tension d'alimentation d'un étage amplificateur ;
- conversion analogique/numérique du signal à une première fréquence d'échantillonnage fe₁ au moins deux fois plus élevée que la première fréquence de coupure fc₁ ;
- traitement de débruitage du signal de fréquence.

## Description

La présente invention se situe dans le domaine de la mesure de données physiologiques de patients par acquisition de signaux électro-physiologiques en temps réel. Par exemple, le contexte peut-être celui de l'acquisition de signaux enregistrant l'activité d'organes humains tels que le cœur, le cerveau etc. Dans le cas spécifique à l'invention, l'acquisition des signaux se fait en outre dans un environnement particulier susceptible de générer des perturbations sur le signal, car elle s'effectue dans un environnement fortement exposé à des champs magnétiques. Ces champs magnétiques résultent par exemple d'un dispositif d'imagerie médicale de type IRM (imagerie à résonance magnétique) auquel est également soumis le patient.

En pratique, étant donné que le principe de ce type d'appareils d'imagerie est basé sur l'application de champs magnétiques non uniformes, effectuée sous forme de séquences d'applications successives de gradients de champ magnétique, ces derniers sont fortement susceptibles de perturber d'autres signaux - comme les électrocardiogrammes (ECG), Electroencéphalogrammes (EEG) etc. - éventuellement recueillis sur un patient soumis au dispositif de génération d'images. C'est précisément l'acquisition de ces signaux qui est au cœur de l'invention, laquelle concerne en fait le traitement à mettre en œuvre pour faire en sorte qu'ils soient utilisables de la manière la plus efficace possible, en l'occurrence débarrassés des perturbations indésirables.

Ces signaux peuvent permettre une surveillance en temps réel du patient pendant les examens IRM, d'où la nécessité de les recueillir avec précision. Alternativement ou additionnellement, ils sont susceptibles d'être employés pour déclencher des opérations de synchronisation de séquences IRM, ou encore pour servir d'appui à d'autres traitements, en lien ou non avec l'IRM. Dans tous les cas, on comprendra que l'obtention de signaux les plus purs possibles, c'est-à-dire débarrassés des bruits parasites qui affectent les mesures brutes, est importante au vu des objectifs mentionnés.

En somme, donc, compte tenu de leur acquisition simultanée au fonctionnement de l'appareillage d'imagerie à résonance magnétique, des interférences entre les signaux que l'on cherche à collecter et les variations magnétiques dues au dispositif imageur sont inévitables, qui brouillent *in fine* le signal recueilli. Elles rendent plus problématique la lecture et l'interprétation du signal mesuré, quelles que soient les finalités fonctionnelles attendues de ce signal. Or, à tout le moins, il faut pouvoir déduire du signal ce qui est nécessaire à la synchronisation des séquences IRM et du monitorage des patients pendant l'examen IRM.

Cette question de la perturbation des signaux de surveillance et de contrôle de type signal ECG, lors de la génération d'images par IRM, est récurrente et apparaît depuis fort longtemps dans la littérature, du fait de son importance pratique dans le domaine de l'imagerie médicale. On a pris conscience depuis les débuts de l'IRM que si le contrôle en continu et en temps réel de l'état du patient est vicié, ou si les opérations de synchronisation et plus généralement les opérations déclenchées par certains états physiologiques mesurés des signaux ne peuvent pas s'effectuer correctement, par manque de précision du signal, cela peut générer des risques dommageables ou simplement priver les mesures de sens.

Il est donc primordial de maîtriser en temps réel non seulement la qualité des signaux recueillis, mais surtout leur fidélité au signal émis que l'on veut mesurer. Ainsi, en reprenant l'exemple du contrôle de l'activité cardiaque d'un patient soumis à une IRM, il est important de recueillir en temps réel un signal d'ECG dont on peut clairement distinguer les artéfacts du signal utile. En d'autres termes, il faut pouvoir distinguer les caractéristiques propres au signal qui pourrait être enregistré en l'absence des aspects liés aux gradients de champs magnétique inhérents au dispositif d'imagerie à résonance magnétique.

L'un des moyens les plus immédiats d'y parvenir est de supprimer lesdits artéfacts. La suppression a en effet pour conséquence de maintenir une correspondance fidèle entre le signal tel que traité pour le débarrasser de ces bruits et le signal produit initialement par l'organe humain. Exprimé autrement, restituer après mesure un signal proche du signal physiologique nécessite d'enlever les bruits enregistrés non directement liés à l'activité par exemple du cœur.

Compte tenu de la prégnance de ce problème dans le temps, de nombreuses solutions ont déjà été proposées pour débarrasser les signaux électro-physiologique, par exemple d'ECG, d'EOG ou d'EEG, de leurs artéfacts par exemple dus aux variations de champ magnétique, parmi lesquelles le positionnement de l'électronique de traitement à proximité du point de mesure et l'utilisation d'une transmission optique ou radiofréquences insensible aux interférences magnétiques. Il a également été proposé l'utilisation de boucles conductrices proches de l'endroit de mesure pour recueillir des signaux identiques aux interférences, qui sont ensuite soustraits aux signaux mesurés. L'idée de mesurer séparément le signal de l'ECG d'une part, et les interférences d'autre part, par un positionnement adapté des capteurs, a d'ailleurs été reprise, mais cette différence de positionnement représente la limite de la solution, puisque par essence, les interférences mesurées ne sont pas exactement celles qui apparaissent à l'endroit de la mesure du signal de l'ECG et que malgré la différence d'emplacement, le second signal mesuré contient aussi une partie du signal utile.

De nombreux procédés basés sur divers traitements des signaux ont par ailleurs été tentés, dont des solutions basées sur le calcul de réponses impulsionnelles aux gradients de champ magnétique, sur la base de la connaissance de leurs courants de génération, pour corriger les artéfacts. Des algorithmes utilisant des méthodes de déconvolution ou des approches adaptatives ont également été proposés.

Pour pallier les limitations de ces solutions, notamment dues aux liaisons électroniques entre le module de traitement du signal et l'électronique de commande du dispositif d'IRM, et qui ne tiennent pas ou que très imparfaitement compte de l'existence de courants de Foucault affectant la précision des calculs, le déposant lui-même a développé une solution, décrite par exemple dans le document WO 2008/155488.

Celle-ci consiste, pour l'essentiel, à combiner le signal de l'ECG à la mesure du champ magnétique local telle que fournie par un capteur par exemple à effet Hall. La mesure des variations temporelles du champ magnétique par un ou plusieurs capteurs de ce type est nécessaire pour à la fois simuler puis supprimer les artéfacts générés dans le signal de l'ECG, par ailleurs mesurés par le ou les capteurs. Pour permettre une correction du signal la meilleure possible, un capteur à effet Hall est - selon cette solution - placé le plus proche possible du capteur de l'ECG, dans l'optique de mesurer les variations de champ magnétique qui provoquent les artéfacts mesurés par ce capteur. La connaissance de ces variations et de leurs conséquences permet un traitement visant à les supprimer en temps réel du signal de l'ECG tel qu'initialement recueilli.

L'invention se situe également dans une perspective de traitement électronique et algorithmique de signaux mesurés en temps réel sur le corps du patient à l'aide de capteurs de signaux physiologiques, et propose une approche simplifiée et efficace de suppression des bruits dans les signaux électro-physiologique qui en résultent.

L'invention concerne tout d'abord un procédé de correction en temps réel d'au moins un signal physiologique mesuré sur un sujet en présence de variations d'au moins une caractéristique d'un environnement électromagnétique telle que les gradients d'un champ magnétique générés par exemple dans un dispositif d'investigation exploitant la résonance magnétique, lesdites variations générant des artéfacts dans ledit signal physiologique, mesuré par des moyens de mesure analogiques solidarisés à au moins une zone de son corps sélectionnée selon le type de signal mesuré, qui est caractérisé par les étapes suivantes :
- filtrage passe-bas du signal mesuré avec une première fréquence de coupure fc₁ supérieure à la fréquence des artéfacts dus aux gradients de champ magnétiques ;
- amplification du signal avec une dynamique d'entrée basée sur une tension égale à au moins 80% de la tension d'alimentation d'un étage amplificateur ;
- conversion analogique/numérique du signal à une première fréquence d'échantillonnage fe₁, ladite première fréquence d'échantillonnage fe₁ étant au moins deux fois plus élevée que ladite première fréquence de coupure fe₁ ;
- traitement de débruitage du signal de fréquence fe₁ par détection des valeurs du signal appartenant aux artéfacts et remplacement desdites valeurs en vue de la suppression des artéfacts.

En substance, selon l'invention, on détecte les artéfacts, ce qui implique de détecter leur début et leur fin, puis on remplace la portion de courbe qui les matérialise par une portion de courbe à leur substituer. Le processus d'interpolation vise de fait à relier en respectant une forme de continuité la portion de courbe de remplacement aux voisinages précédant ou suivant immédiatement les deux bornes identifiées desdites courbes des artéfacts. Mais cela n'est possible que si les conditions initiales posées ci-dessus sur les fréquences de coupure et d'échantillonnage sont respectées, permettant une bande passante suffisante pour ne pas supprimer, dans le signal à traiter, les artefacts dus aux gradients. La forte dynamique d'entrée est par ailleurs nécessaire pour acquérir et traiter les très grandes différences d'amplitude entre les parties du signal dues aux artéfacts et celles qui relèvent des variations physiologiques, comme on le verra plus en détail dans la suite. Enfin, il est à noter que, dans le cadre du procédé selon l'invention, il n'y a jamais de filtrage passe-haut, et que la composante continue du signal est par conséquent autant que possible gardée.

Plus précisément, le traitement de débruitage comporte les étapes suivantes :
- analyse permanente des valeurs du signal mesuré en vue de détecter leur appartenance aux artéfacts ;
- si une valeur mesurée est considérée comme appartenant à un artéfact, remplacement de ladite valeur par une valeur calculée en vue de l'interpolation d'une nouvelle portion de courbe en lieu et place d'un artéfact dans la courbe formée par les valeurs successives numérisées du signal mesuré.

La détection de l'appartenance aux artéfacts des points de signaux mesurés s'effectue en pratique, selon une configuration possible de l'invention, en prenant en compte le fait que les formes desdits artéfacts sont très différentes de l'aspect usuel du reste du signal ECG normal : il s'agit de pics d'amplitudes positive et négative très supérieure aux autres variations du signal, qui peuvent atteindre jusqu'à 400 fois l'amplitude des variations propres à l'ECG, voire plus. C'est pour cette raison qu'il est nécessaire que l'amplification dispose d'une très grande dynamique d'entrée, pour empêcher toute saturation du signal en sortie. Ces pics extrêmement aigus se caractérisent par des pentes, de part et d'autre des points extrêmes de réversion, très importantes. La pente peut donc être vue comme un marqueur qui caractérise de façon très fiable les artéfacts.

Par conséquent, la détection de l'appartenance des portions de signal aux artéfacts peut être réalisée par mesure de la pente du signal en tout point et comparaison avec une valeur de pente seuil. Encore plus précisément, de préférence, la détection de la pente en un point s'effectue par mesure de la dérivée en ce point et calcul de sa valeur absolue. Le système de l'invention, en l'espèce matérialisé par un dispositif de traitement du signal du type microprocesseur, microcontrôleur, DSP, FPGA ou ordinateur, qui sera envisagé plus en détail dans la suite, stocke en mémoire au moins une valeur seuil de pente de courbe par rapport à laquelle la comparaison est effectuée après chaque calcul de pente. Lorsque la comparaison renvoie un résultat positif, c'est-à-dire lorsque la pente mesurée est supérieure à la valeur seuil, le système considère que le point appartient à un artéfact. Les opérations d'interpolation sont alors mises en œuvre pour la substitution de points ou valeurs dans la courbe.

Ladite interpolation peut, selon une possibilité de mise en œuvre, comporter les étapes suivantes :
- analyse de la valeur instantanée du signal mesuré ;
- analyse de n valeurs précédentes du signal ;
- réalisation d'une moyenne des n valeurs ; et
- remplacement de la valeur instantanée par la moyenne des n valeurs précédentes.

Cette opération n'intervient bien entendu que pour les valeurs ou les points identifiés comme faisant partie d'un artéfact à supprimer de la courbe du signal électro-physiologique mesuré en temps réel.

En somme, l'analyse en continu des valeurs mesurée par les capteurs analogiques ensuite converties en un codage numérique, notamment à l'aune d'un algorithme de détection de pente, permet de valider ou non leur appartenance à un artéfact. Dans l'hypothèse d'une validation, un algorithme d'interpolation permet de remplacer les valeurs des artéfacts par des valeurs plus proches de la courbe naturelle par exemple d'un ECG.

Comme on l'a vu, le procédé de l'invention implémente une double opération d'échantillonnage/filtrage, d'abord en début de procédé à partir des signaux électro-physiologiques analogiques obtenus via les moyens capteurs, dans le but de pouvoir réaliser un traitement électronique sur des signaux numérisés, puis en fin de procédé pour un dernier filtrage du signal expurgé qui rend la courbe finale proche de celle obtenue par un enregistrement dans un contexte dépourvu de perturbations électro-magnétiques trop intenses.

De fait, selon l'invention, l'objectif est de pouvoir acquérir le signal ECG et en même temps le signal parasite issu des gradients de champ magnétique (les artéfacts dans le signal physiologique). Ainsi, comme la fréquence des artéfacts est en pratique de l'ordre de 500Hz à 10kHz, entraînant une perturbation sur le signal physiologique d'ECG de même fréquence, le premier filtrage passe-bas à la première fréquence de coupure fc₁ doit couper au-delà de cette fréquence des artéfacts dus aux gradients pour réduire au maximum l'atténuation de ce signal parasite, qui n'est pas recherchée.

La fréquence de coupure fc₁ est alors choisie entre 20 kHz et 256 kHz, de préférence entre 20 kHz et 150 kHz pour faire une acquisition avec une plus grande précision en vue du traitement du signal, en gardant toute l'information dudit signal.

La première fréquence d'échantillonnage fe₁ doit idéalement, comme mentionné auparavant, être supérieure au double de la fréquence des artéfacts des signaux mesurés. Elle est ainsi choisie pour être comprise entre 10kHz et 40 kHz, de préférence de l'ordre de 16 kHz. Cette valeur préférentielle de 16 kHz permet de garder de façon précise le début et la fin d'un artéfact.

En sortie, pour lisser en quelque sorte le signal électro-physiologique, après le traitement visant à supprimer les artefacts de gradient de champ magnétique, un second filtrage passe-bas avec une seconde fréquence de coupure fc₂ est appliqué, pour permettre ensuite un sous-échantillonnage du signal débruité à une seconde fréquence d'échantillonnage fe₂. Ladite seconde fréquence d'échantillonnage fe₂ peut être comprise entre 250Hz et 2kHz, et de préférence être de l'ordre de 1 kHz. La seconde fréquence de coupure fc₂ est comprise entre 20 Hz et 150 Hz, de préférence de l'ordre de 120 Hz.

Selon l'invention, on a indiqué qu'il est indispensable de conserver autant que possible et de la meilleure manière la valeur continue du signal (DC) avant la conversion analogique/numérique. Elle n'est donc pas filtrée. Un filtre passe-haut qui aurait pour but de supprimer la valeur continue (offset) aurait en effet aussi pour conséquence de déformer le signal et rendre plus difficile le traitement ultérieur. Dans la présente invention, aucun filtrage passe-haut n'est donc appliqué avant la conversion analogique/numérique.

Pour permettre l'acquisition de signaux à haute fréquence, ce qui est réalisé avant tout traitement des signaux visant à les débarrasser des artéfacts, et conserver la valeur de l'offset du signal, il faut aussi veiller à ne pas saturer le signal. Dans la présente invention, comme déjà mentionné, la dynamique de l'amplification avant la conversion analogique/digitale doit être très grande à la fois pour garder une résolution suffisante pour le signal ECG et également pour ne pas saturer les artéfacts de gradients de champ magnétique ayant une amplitude qui peut aller jusqu'à 400 fois celle des éléments du signal physiologique d'ECG.

Ce procédé de l'invention, applicable en tant que tel dans le contexte évoqué en ouverture, peut par ailleurs aussi être associé à d'autres procédés existants donnant lieu à enregistrements de signaux comportant des artéfacts à forte pente, qu'il peut alors contribuer à améliorer.

Outre le procédé dont les caractéristiques sont mentionnées ci-dessus, l'invention a également trait à un dispositif correspondant, permettant de débarrasser de leurs artéfacts des signaux électro-physiologiques mesurés chez un patient - toujours dans le contexte identifié auparavant.

Plus précisément, l'invention couvre alors un dispositif de correction en temps réel d'un signal électro-physiologique mesuré sur un sujet en présence de variations d'au moins une caractéristique d'un environnement électromagnétique existant par exemple dans un dispositif d'investigation exploitant la résonance magnétique, caractérisé en ce qu'il comporte :
- Des moyens capteurs analogiques du signal solidarisés à une zone du corps du sujet dépendant du type de signal mesuré ;
- un module électronique d'amplification et de conversion analogique/numérique du signal physiologique issu des moyens capteurs à une première fréquence d'échantillonnage fe₁ incluant un premier filtrage passe-bas à une première fréquence de coupure fc₁ ;
- un étage de traitement des signaux relié à l'étage d'amplification et de conversion analogique/numérique ;
- un module de liaison insensible aux perturbations électromagnétiques reliant l'étage de traitement et l'entrée d'une unité de traitement standard de type ordinateur apte à faire fonctionner un logiciel de traitement des données.

En substance, la mesure initiale des données s'effectue de manière analogique, par les moyens capteurs qui sont par exemple fixées sur la poitrine du patient dans l'hypothèse d'un ECG. Puis, l'étage électronique réalise une amplification, la conversion numérique et un filtrage numérique des signaux. Ces signaux numérisés sont ensuite envoyés à l'étage de traitement des signaux, par exemple un microprocesseur, ou un microcontrôleur, ou un processeur spécialisé dans le traitement du signal type DSP, ou des circuits intégrés reprogrammables de type FPGA et/ou un ordinateur situé à distance et sur lequel un programme standard de traitement de signaux peut le cas échéant être implémenté.

L'un des problèmes pratiques déjà mentionné qui se posent dans un tel système réside dans le fait que l'amplitude des signaux correspondant aux artéfacts est très élevée, et peut par exemple atteindre jusqu'à près de 400 fois l'amplitude du signal de l'ECG proprement dit. Il est donc nécessaire de disposer d'une électronique dotée d'une très grande dynamique d'entrée, faute de quoi l'inévitable saturation de l'étage d'entrée pourrait avoir comme effet une inversion du signal ou un comportement inapproprié de l'étage en question. De ce fait, le module d'amplification et de conversion, qui comporte des moyens d'amplification de type amplificateurs opérationnels, est conçu pour une forte dynamique d'entrée, basée sur une tension de l'ordre de 80% de la tension d'alimentation du module.

Selon une configuration possible, les moyens capteurs peuvent comporter au moins deux électrodes de surface et une électrode de référence à conducteur neutre qui peut avantageusement améliorer le signal. Les conducteurs issus desdites électrodes sont reliés à l'étage électronique d'amplification et de conversion. Dans la présente invention, 4 électrodes sont utilisées, incluant 3 électrodes de mesure et une électrode de réduction des interférences de mode commun.

Le dispositif de l'invention fournit par conséquent 3 signaux d'ECG indépendants du même patient objets d'analyse en parallèle par les étages ultérieurs dudit dispositif.

On a mentionné auparavant le caractère d'insensibilité aux perturbations électromagnétiques, qui est particulièrement important et requis dans un environnement tel que celui de l'exemple préférentiel autour duquel est articulée la présente description, à savoir une mesure de signaux électro-physiologiques concomitante à la mise en œuvre d'opérations d'imagerie médicale de type IRM. Dans ce contexte, et selon une possibilité, le module de liaison peut consister en un module de raccordement à fibres optiques, lesquelles présentent précisément une telle insensibilité.

L'invention va à présent être décrite plus en détail, en référence aux figures annexées qui illustrent un exemple non limitatif de mise en œuvre de l'invention, dans l'exemple spécifique de la mesure d'ECG, et pour lesquelles :
- la figure 1 représente très schématiquement l'architecture du dispositif de l'invention ;
- la figure 2 montre une possibilité d'implantation des électrodes de mesure des signaux d'ECG sur un sujet soumis à investigations ;
- la figure 3 illustre de façon schématique un schéma électronique de circuit d'amplification et de conversion/filtrage des signaux analogiques issus des électrodes de mesure ;
- la figure 4 montre les diagrammes temporels synchronisés des trois signaux d'ECG convertis et des signaux débruités selon le procédé de l'invention ;
- la figure 5 illustre la succession des étapes mises en œ uvre sur une portion de signal mesuré ;
- la figure 6 montre la même succession de diagrammes qu'en figure 4, en fin de procédé lorsque les signaux d'ECG sont complètement expurgés des artéfacts, après échantillonnage et filtrage final ; et
- la figure 7 représente schématiquement une opération d'interpolation telle que mise en œuvre par le procédé de l'invention dans l'étape de débruitage.

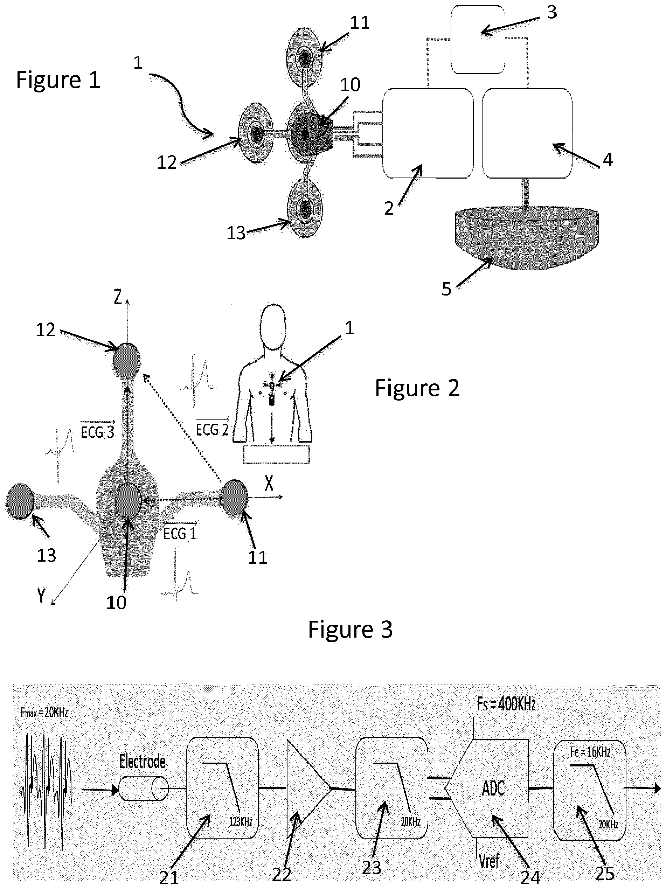

En référence tout d'abord à la figure 1, le dispositif dans son ensemble se compose de moyens capteurs, par exemple un « patch » ECG (support d'électrodes) permettant le recueil des signaux physiologiques d'ECG, par lesquels lesdits signaux électro-physiologiques sont mesurés de manière analogique. Un module 2 spécifique au traitement des signaux d'ECG est pourvu en aval des électrodes de mesure 10, 11, 12, 13. Il s'agit en fait d'un dispositif classique recueillant les signaux d'ECG et apte à les transformer en vue de traitements ultérieurs. Les signaux ECG1, ECG2, ECG3 transmis par les électrodes 10, 11 et 12 sont analogiques et amplifiés puis convertis en des signaux numériques par exemple par les étages électroniques successifs tels qu'ils apparaissent en figure 3, avant d'être envoyés à un micro-contrôleur 3. Les conducteurs en entrée du module de conversion 2 véhiculent donc des signaux analogiques alors que les liaisons en traits pointillés entre ladite interface 2 et le micro-contrôleur 3 d'une part, puis en sortie de celui-ci vers un module de raccordement à fibre optique 4 d'autre part sont parcourus par un signal numérique échantillonné par exemple à 16 kHz. La portion en fibre optique en aval du module 4, insensible aux perturbations électromagnétiques présentes dans l'environnement d'un imageur IRM, véhicule le signal numérisé vers une entrée série d'un ordinateur classique 5. Les éléments 2, 3 et 4 sont placés dans une cage de Faraday. Un filtre de passage 21 (voir en figure 3) est dès lors prévu entre les signaux des électrodes et l'entrée de l'amplificateur/convertisseur 2.

La figure 2 rappelle que dans l'hypothèse de l'enregistrement d'un signal d'ECG, les électrodes 10 à 13 formant les moyens capteurs des signaux cardiaques sont à l'évidence attachées sur la poitrine du patient au regard/ au voisinage du cœur.

La figure 3 montre de manière plus détaillée le traitement électronique d'amplification et de filtrage appliqué aux signaux analogiques recueillis au niveau des électrodes 11 à 13, en vue de leur conversion en signaux numériques avant débruitage ultérieur. Ce premier traitement est réalisé dans le module 2 visible en figure 1. L'amplification se faisant dans une cage de Faraday, il est nécessaire d'intégrer au circuit un filtre analogique passe-bas 21 indispensable pour garder la protection contre les ondes électromagnétiques radiofréquences (64 ou 128 MHz) de la cage de Faraday. Ce filtre 21 doit filtrer la radiofréquence mais garder les informations ECG et de gradient de champ magnétique. Il est prévu avec une fréquence de coupure de l'ordre de 123 kHz, de sorte que les fréquences utiles du signal d'ECG sont conservées mais pas les radiofréquences. Ce filtrage constitue également le filtre anti-repliement avant un amplificateur 22 et la partie de conversion analogique/numérique 24 proprement dite, séparés par un filtre anti-aliasing 23 qui permet aussi le passage des signaux perturbateurs des gradients de champ magnétique. Les deux étages de filtrage précités 21, 23 sont prévus tels que le filtrage opéré réalise la première fréquence de coupure fc₁ au sens de l'invention, ici de l'ordre de 20 kHz.

Un premier échantillonnage des signaux d'électrocardiogramme peut se faire par exemple à 400 kHz, avant un sous-échantillonnage final à environ 16 kHz, qui représente la première fréquence d'échantillonnage fe₁ au sens de l'invention. Cette fréquence d'échantillonnage doit être, comme on l'a vu, égale à au moins deux fois les fréquences des artéfacts que laisse passer le filtrage passe-bas réalisé en amont par les filtres 21,23.

Ce ne sont là que des exemples possibles, les valeurs citées n'étant nullement limitatives. Le traitement de signal peut se faire directement dans le microcontrôleur 3 (voir en figure 1) ou dans l'ordinateur distant 5. Dans le cas d'un traitement de signal local, il est ensuite possible de sous-échantillonner le signal à une fréquence inférieure à 16Khz pour faciliter le transfert des informations à l'ordinateur. Dans le cas d'un traitement de signal dans l'ordinateur, le signal complet doit être transmis. Malgré la pluralité des filtrages déjà intervenus à ce stade, les artéfacts ne sont pas supprimés en sortie de l'interface 2, pour permettre de les traiter efficacement.

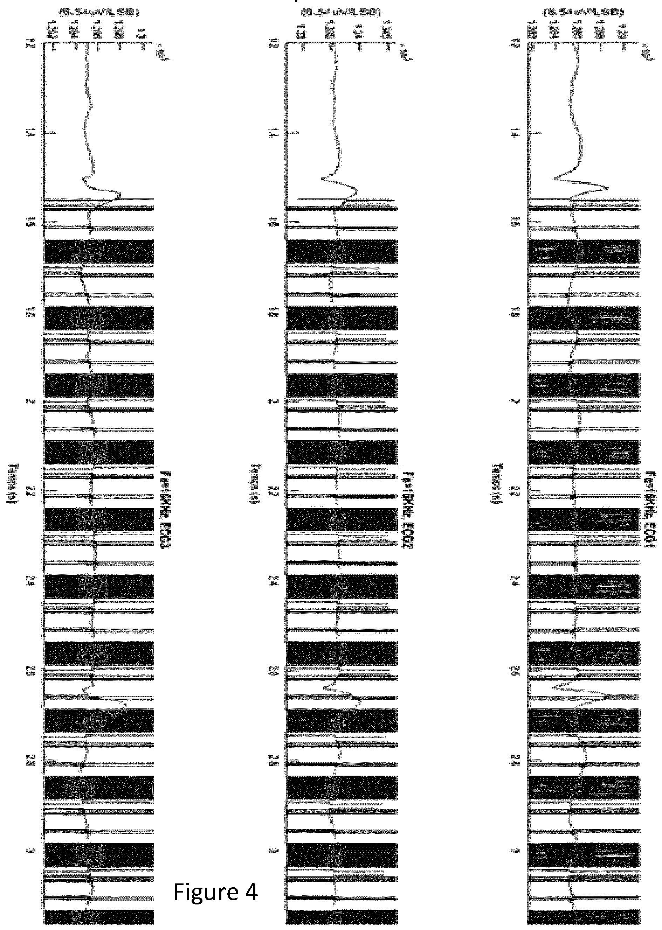

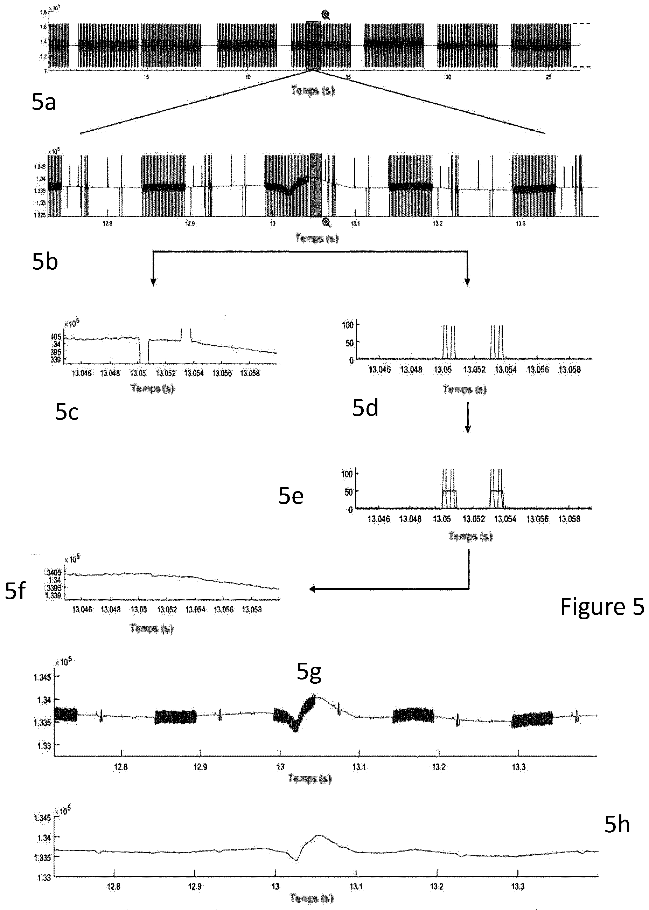

Les signaux ECG1, ECG2, ECG3 numérisés obtenus en sortie de cette interface 2 ont la forme qui apparaît en figure 4, dans laquelle chaque diagramme contient deux signaux distincts superposés : des signaux représentés en noir d'amplitude maximale correspondent à ceux qui sortent « bruts » de l'interface 2 de la figure 1, et des signaux centraux représentés en gris qui ne présentent plus que des reliquats très atténués des artéfacts, après que l'algorithme de débruitage ait été mis en œuvre. La figure 5 détaille le processus de débruitage en se focalisant sur une fenêtre temporelle 51 de faible durée du signal numérisé non débruité obtenu en sortie de l'interface 2, comme cela apparaît dans le diagramme supérieur 5a. Cette fenêtre est agrandie dans le second diagramme 5b, placé juste en dessous, qui montre la même superposition de signaux, bruts et débruités, que dans le diagramme de la figure 4, sur une durée bien moindre. Une nouvelle fenêtre temporelle 52 d'échelle temporelle encore beaucoup plus limitée en est extraite pour les besoins de l'explication du fonctionnement du procédé de débruitage. Le signal brut issu de cette fenêtre 52 est montré en le diagramme 5c, comportant en l'occurrence deux artéfacts. Ce signal est ensuite dérivé, et on conserve la valeur absolue de la dérivée pour obtenir le signal du diagramme 5d. Les valeurs de ce signal dérivé de 5d sont par la suite soumises à un traitement de suppression des artéfacts dont on donnera un exemple dans la suite de sorte que seul le signal expurgé apparaissant sur le diagramme 5f subsiste.

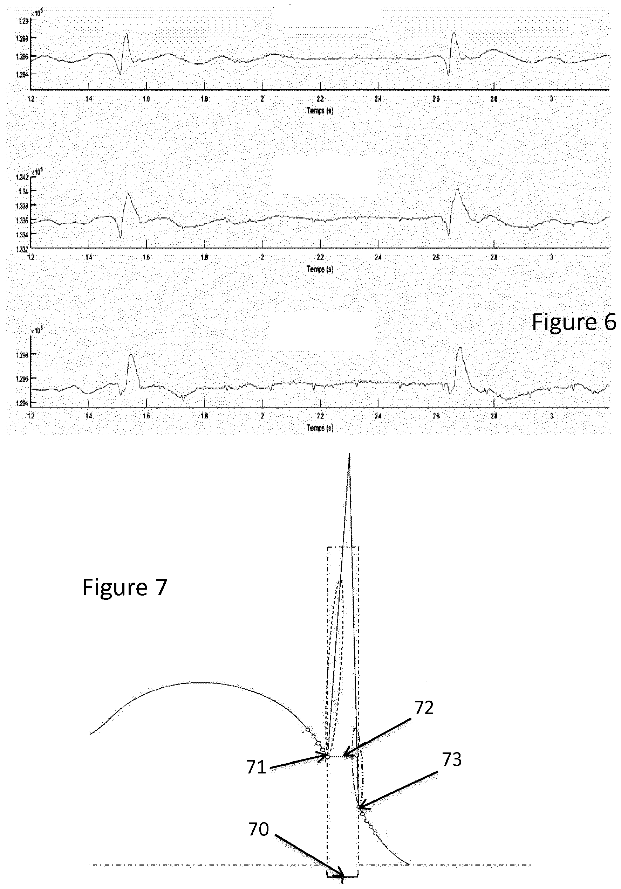

Si l'on effectue ce traitement de suppression des artéfacts sur l'ensemble du signal de la fenêtre 51, dont le diagramme est développé en 5b, cela donne une courbe de signal telle que celle qui apparaît en figure 5g. La réalisation de l'échantillonnage final à fréquence fe₂ accompagnée du filtrage à seconde fréquence de coupure fc₂ donne enfin lieu au signal final montré en 5h, qui apparaît comme correctement débarrassé des artéfacts, suite au traitement global de l'invention. Ce résultat final, pour les trois signaux issus des trois électrodes 10, 11 et 12, est également celui qui est montré en figure 6.

Un exemple de traitement simplifié de suppression des artéfacts par interpolation d'une courbe de remplacement est illustré par la figure 7. Dès la détection d'un artéfact par analyse d'une valeur instantanée de point, au moyen de l'algorithme de détection de pente comportant par exemple l'étape de dérivation mentionnée et une étape de comparaison avec une pente seuil, c'est-à-dire dès le point 71 à l'entame de la fenêtre de remplacement de valeurs 70, l'interpolation se met en place. Le point 71, après analyse, est détecté comme faisant partie d'un artéfact du fait de sa pente élevée : les n valeurs précédentes (par exemple les quatre valeurs précédentes) sont alors utilisées pour calculer une valeur de remplacement, par exemple par un traitement de moyennage. Il en va ainsi pour toutes les valeurs suivantes de la fenêtre 70 dont l'analyse instantanée montre qu'elles font partie d'un artéfact. Elles sont toutes remplacées suivant un algorithme d'interpolation de valeurs par exemple sur le modèle ci-dessus. En généralisant, on peut que toutes les valeurs v_{N} à l'instant N sont dans ce cas remplacées par des valeurs v_{N-1} basées sur des calculs effectués à l'étape précédente, et qui prennent donc en compte la valeur déjà interpolée à N-1. Les valeurs extrapolées 72 prennent donc place à l'intérieur d'un pic constitué par l'artéfact, entre la valeur d'entrée 71 et une valeur de sortie 73 toujours détectée au moyen de l'algorithme de détection mentionné ci-dessus. La courbe, débarrassée des pics d'artéfact, est lissée pour s'approcher de la forme du signal d'ECG qu'on obtiendrait en l'absence de gradients de champ magnétique.

L'exemple d'un électrocardiogramme a constitué le fil conducteur de la description ci-dessus, mais il est à noter que la possibilité offerte par l'invention d'une suppression claire, dans les signaux obtenus, des différents artéfacts, peut aussi s'appliquer à d'autres types de signaux du fait des caractéristiques du procédé, qui ne sont pas liées à une typologie de signaux mais sont basées sur du traitement du signal apte à s'abstraire de l'origine et de la nature de celui-ci.

L'invention ne se limite bien entendu pas aux exemples décrits et expliqués en référence aux figures, mais elle englobe les variantes et versions de forme et de conception applicables aux éléments réalisant les mêmes fonctions que dans l'invention.

## Revendications

1. Procédé de correction en temps réel d'au moins un signal électro-physiologique mesuré sur un sujet en présence de variations d'au moins une caractéristique d'un environnement électromagnétique telle que les gradients d'un champ magnétique générés par exemple dans un dispositif d'investigation exploitant la résonnance magnétique, lesdites variations générant des artéfacts dans ledit signal électro-physiologique, mesuré par des moyens de mesure analogiques solidarisés à au moins une zone de son corps sélectionnée selon le type de signal mesuré, **caractérisé par** les étapes suivantes :
- filtrage passe-bas du signal mesuré avec une première fréquence de coupure fc₁ supérieure à la fréquence des artéfacts dus aux gradients de champ magnétique ;
- amplification du signal avec une dynamique d'entrée basée sur une tension égale à au moins 80% de la tension d'alimentation d'un étage amplificateur ;
- conversion analogique/numérique du signal à une première fréquence d'échantillonnage fe₁, ladite première fréquence d'échantillonnage fe₁ étant au moins deux fois plus élevée que ladite première fréquence de coupure fe₁ ;
- traitement de débruitage du signal de fréquence fe₁ par détection des valeurs du signal appartenant aux artéfacts et remplacement desdites valeurs en vue de la suppression des artéfacts.

2. Procédé de correction en temps réel d'un signal électro-physiologique selon la revendication précédente, **caractérisé en ce que** le traitement de débruitage comporte les étapes suivantes :
- analyse permanente des valeurs v_{N} du signal mesuré en vue de détecter leur appartenance aux artéfacts ;
- si une valeur v_{N} mesurée est considérée comme appartenant à un artéfact, remplacement de ladite valeur par une valeur calculée en vue de l'interpolation d'une nouvelle portion de courbe en lieu et place d'un artéfact dans la courbe formée par les valeurs successives numérisées v_{N} du signal mesuré.

3. Procédé de correction en temps réel d'un signal électro-physiologique selon la revendication précédente, **caractérisé en ce que** la détection de l'appartenance des portions de signal aux artéfacts est réalisée par mesure de la pente du signal en tout point v_{N} et comparaison avec une valeur de pente seuil.

4. Procédé de correction en temps réel d'un signal électro-physiologique selon la revendication précédente, **caractérisé en ce que** la détection de la pente en un point v_{N} s'effectue par mesure de la dérivée en ce point v_{N} et calcul de sa valeur absolue.

5. Procédé de correction en temps réel d'un signal électro-physiologique selon l'une des revendications 2 à 4, **caractérisé en ce que** l'interpolation comporte les étapes suivantes :
- analyse de la valeur instantanée v_{N} du signal mesuré ;
- analyse des n valeurs précédentes du signal ;
- réalisation d'une moyenne des n valeurs ; et
- remplacement de la valeur instantanée v_{N} par la moyenne des n valeurs précédentes.

6. Procédé de correction en temps réel d'un signal électro-physiologique selon l'une des revendications précédentes, **caractérisé en ce que** la première fréquence de coupure fc₁ est comprise entre 20 kHz et 256 kHz, de préférence entre 20 kHz et 150 kHz.

7. Procédé de correction en temps réel d'un signal électro-physiologique selon l'une des revendications précédentes, **caractérisé en ce que** la première fréquence d'échantillonnage fe₁ est comprise entre 10kHz et 40 kHz, de préférence de l'ordre de 16 kHz.

8. Procédé de correction en temps réel d'un signal électro-physiologique selon l'une des revendications précédentes, **caractérisé par** un second filtrage passe-bas avec une seconde fréquence de coupure fc₂ pour permettre un sous-échantillonnage du signal débruité à une seconde fréquence d'échantillonnage fe₂.

9. Procédé de correction en temps réel d'un signal électro-physiologique selon la revendication précédente, **caractérisé en ce que** la seconde fréquence d'échantillonnage fe₂ est comprise entre Hz 250 Hz et 2 kHz, de préférence de l'ordre de 1 kHz.

10. Procédé de correction en temps réel d'un signal électro-physiologique selon la revendication précédente, **caractérisé en ce que** la seconde fréquence de coupure fc₂ est comprise entre 20 Hz et 150 Hz, de préférence de l'ordre de 120 Hz.

11. Dispositif de correction en temps réel d'un signal électro-physiologique mesuré sur un sujet en présence de variations d'au moins une caractéristique d'un environnement électromagnétique existant par exemple dans un dispositif d'investigation exploitant la résonance magnétique, **caractérisé en ce qu'**il comporte :
- des moyens capteurs analogiques (1) du signal solidarisés à une zone du corps du sujet dépendant du type de signal mesuré ;
- un module électronique d'amplification et de conversion analogique/numérique (2) du signal physiologique issu des moyens capteur à une première fréquence d'échantillonnage fe₁ incluant un premier filtrage passe-bas à une première fréquence de coupure fc₁ ;
- un étage de traitement des signaux (3) relié au module électronique d'amplification et de conversion analogique/numérique (2) ;
- un module de liaison (4) insensible aux perturbations électromagnétiques reliant l'étage de traitement (3) et l'entrée d'une unité de traitement standard, par exemple un ordinateur (5) apte à faire fonctionner un logiciel de traitement des données.

12. Dispositif de correction en temps réel d'un signal électro-physiologique selon la revendication précédente, **caractérisé en ce que** le module d'amplification et de conversion (2) comporte des moyens d'amplification (22), par exemple un amplificateur opérationnel à forte dynamique d'entrée basée sur une tension de l'ordre de 80% de la tension d'alimentation du module (2).

13. Dispositif de correction en temps réel d'un signal électro-physiologique selon l'une des revendications 11 et 12, **caractérisé en ce que** les moyens capteurs (1) comportent au moins deux électrodes de surface (10, 11, 12) et une électrode de référence à conducteur neutre (13), les conducteurs issus desdites électrodes (10 à 13) étant reliés à l'étage électronique d'amplification et de conversion (2).

14. Dispositif de correction en temps réel d'un signal électro-physiologique selon la revendication précédente, **caractérisé en ce que** les moyens capteurs (1) comportent trois électrodes de mesure (10, 11, 12,), et une électrode (13) de réduction des interférences de mode commun.

15. Dispositif de correction en temps réel d'un signal électro-physiologique selon la revendication précédente, **caractérisé en ce que** le module de liaison (4) consiste en un module de raccordement à fibres optiques.
